Europäisches Patentamt

(19) European Patent Office

Office européen des brevets

(11) Publication number: **0 326 884 B1**

# EUROPEAN PATENT SPECIFICATION

(45) Date of publication of patent specification:
06.11.91 Bulletin 91/45

(51) Int. Cl.⁵ : **A61K 7/22**

(21) Application number : **89101059.7**

(22) Date of filing : **21.01.89**

(54) Formulations for the oral hygiene.

(30) Priority : **01.02.88 IT 1926488**

(43) Date of publication of application :
**09.08.89 Bulletin 89/32**

(45) Publication of the grant of the patent :
**06.11.91 Bulletin 91/45**

(84) Designated Contracting States :
**AT BE CH DE FR GB LI LU NL SE**

(56) References cited :
**US-A- 4 024 237**
**US-A- 4 213 961**

(56) References cited :
**CHEMICAL ABSTRACTS, vol. 79, 1973, page 98, abstract no. 74282w, Columbus, Ohio, US; M. KOSUGE: "Effect of sucrose on the solubility of teeth", & KAKU EISEI GAKKAI ZASSHI 1972, 22(3), 325-39**

(73) Proprietor : **Campo, Giovanni**
**Largo Corsia dei Servi, 11**
**I-20122 Milano (IT)**

(72) Inventor : **Campo, Giovanni**
**Largo Corsia dei Servi, 11**
**I-20122 Milano (IT)**

(74) Representative : **Minoja, Fabrizio**
**Studio Consulenza Brevettuale Via Rossini, 8**
**I-20122 Milano (IT)**

## Description

The invention refers to formulations useful for oral hygiene for the prevention of the dental plaque and of caries as well as co-adjuvants in the prevention and cure of the common diseases of oral mucosa and of gums. The formulations of the invention contain as an active principle tromethamol (mono-tris(hydroxymethyl)amino methane) combined with pectic acid or polygalacturonic acid. Because of the basic and acid characteristics of the two substances, a salt will be formed, at least partially. The invention comprises in any way all the possible forms in which said combination may be presented (complex, combination, salt with not defined stoichiometry, etc.). The molar ratio tromethamol/pectic acid is from preferably 0.5 to 1.5 and it is most preferably about 1 : 1. The presently known formulations used for the prevention and treatment of the dental plaque generally rely on the use of antimicrobial agents. See for instance US Patent 4213961 ; US Patent 4024237 discloses oral compositions characterized by more components, one of which is selected in the group consisting of dextrane, heparin or other polyanionic polysaccharides as agents able to mantain a colloidal and viscous environment.

The use of tromethamol as buffering agent is known and it is disclosed namely in odontosthomalogic field itself in said US Patents and in Chem. Abstr. 74282 w, Vol. 79, 1973, p. 98. Tromethamol is a known aminic base characterized by a masked buffering activity and already used in therapy in the systemic treatment of acidosic states (gastric hypersecretion). It is also known the capacity of tromethamol of entering the intra- and extracellular compartments.

The preventive and therapeutic activity of tromethamol is surprisingly enhanced by the combination with pectic acid, known component of vegetal pectins ; it is known to be endowed with hemostatic, cytoprotective and antibacterial properties. It is also known the calcium scavenging action of pectic acid.

It has been in fact found that the combination tromethamol-pectic acid is able to hinder the occurrence of caries and of the dental plaque and it is useful in the prevention and cure of the common diseases of the oral mucosa generally, and of the gums in particular (ulcerations, stomatitis, gengivitis, parodontopathies, etc.).

The formulations of the invention containing pectic acid and tromethamol have the following advantages and properties :

— synergistic anticaries properties ;
— ability of coming into contact also with the cellular interstices ;
— prolonged effect in time thanks to the viscosity of the obtained solutions ;
— absorption ability of harmful bacterial flora because of the "in vivo" formation of free pectic acid ;
— removal of calcium ions which are no more available for the formation of the dental plaque through bridges with positive charged glycoproteins inclining to fix to the negative charges of enamel ;
— manteinance of the physiological pH, essential to prevent lack of balance between the normal bacterial flora and the cariogenic one and diseases of the oral mucosa (gengivitis) ;
— better practicality in the pharmaceutical preparation, because of the water solubility of the combination, salt or complex.

The formulations of the invention contain from 0.01 to 10% by weight of tromethamol or the equivalent of a salt thereof with non-toxic organic or inorganic salts such as hydrochloric, phosphoric, carbonic, acetic, malic, citric, maleic, salicylic, acetylsalicylic acid. As pectic acid, it may be used, for instance, that commercially available (Fluka, No. 76300). The compositions of the invention will contain also conventional vehicles or excipients and possibly other active principles having complementary or anyhow useful activity such as allantoine or allantoinates, vegetal extracts, vitamins, antibiotics, disinfectants, hemostatics, astringents, antimycotics, fluorine compounds, abrasives, bleaching agents, surfactants, non cariogenic sugar such as xilitol or maltitol.

According to the invention it is possible to provide all the range of the already known forms used in the care and hygiene of teeth and mouth : tooth-past (in powder, liquid or paste form), gingival washings, mouth washes (liquid or for extemporaneous use), gengidentrifices, gingival preparations. Said formulations will be used in the daily hygiene of the oral cavity twice or more times a day, generally after meals. In the case of liquid forms, about 50 ml of formulation for each application will be used.

The following examples further illustrate the invention.

## EXAMPLE 1

### MOUTH WASHES

| A) Tromethamol | g | 1 |
| Pectic acid | g | 1 |
| Water | ml | 500 |

| B) Tromethamol | g | 0,6 |
| Pectic Acid | g | 1 |
| HCl (M 0.1) | ml | 40 |
| Distilled water q.s. to | ml | 300 |

A buffer solution at pH 7.5 is obtained.

| C) Maleate tromethamol acid | g | 2.3 |
| Pectic acid | g | 2.0 |
| NaOH 0.2 M | ml | 48 |
| Distilled water q.s. to | ml | 500 |

A buffer solution at pH is obtained.

| D) Tromethamol | g | 1 |
| Pectic acid | g | 1.2 |
| Allantoine | g | 0.5 |
| Maltitol | g | 10 |
| NaF | g | 0.1 |
| Cetyl trimethylammonium p-toluenesulfonate | g | .0.1 |
| Peppermint flavour | g | 0.1 |
| Water q.s. to | ml | 500 |

## EXAMPLE 2

### POWDER FORMULATIONS FOR EXTEMPORANEOUS USE

| | | |
|---|---|---|
| Tromethamol | g | 10 |
| Pectic acid | g | 13 |
| Maltitol | g | 100 |
| Allantoine | g | 5 |
| NaF | g | 1 |
| Cetyltrimethylammonium p-toluenesulfonate | g | 1 |
| Powder peppermint flavour | g | 1 |

The mixture is throrougly mixed and the sieved through a fine mesh. The formulation is distributed in thermo-sealed alluminium envelopes containing 1.2 g each. The content of an envelope is dissolved in half glass (about 50 ml) of water before use.

## EXAMPLE 3

### TOOTH-PASTE

| | | |
|---|---|---|
| A) Tromethamol | g | 10 |
| Pectic acid | g | 8 |
| Caboxymethylcellulose | g | 6 |
| Maltitol | g | 30 |
| Sodim laurylsulfate | g | 1 |
| Colloidal silica | g | 1.5 |
| Allantoine glicyrrethic acid complex | g | 1 |
| Powder liquorice extract | g | 0.1 |
| Preserved water q.s. to | g | 100 |

| B) Tromethamol | g | 15 |
|---|---|---|
| Pectic acid | g | 20 |
| Proteinate silver | g | 1 |
| Sodiumlaurylsulfate | g | 1.5 |
| Calcium carbonate | g | 30 |
| Hydroxyethylcellulose | g | 1 |
| Ixantan gum | g | 0.5 |
| Glicerine | g | 25 |
| Propoli tincture | g | 4 |
| Ratania tincture | g | 5 |
| Preserved water q.s. to | g | 100 |

## EXAMPLE 4

### GINGIVAL PASTE

| Tromethamol | g | 1 |
|---|---|---|
| Pectic acid | g | 1.5 |
| Allantoine | g | 0.5 |
| Carboxymethylcellulose average viscosity | g | 6 |
| Colloidal silicas | g | 1 |
| Powdered strawberry flavour | g | 0.1 |
| E 124 color | g | 0.01 |
| Water q.s. to | g | 100 |

## EXAMPLE 5

| Tromethamol | g | 121 |
|---|---|---|
| Pectic acid | g | 173 |

The mixture is thorougly mixed in a ball-mill for about 5 hours. The so obtained powder is used as "active principle" in the following preparations.

A) Mouth washes

| | | |
|---|---|---|
| Active principle | g | 3 |
| Peppermint flavour | g | 0.1 |
| Preserved water q.s. to | g | 500 |

B) Powder formulation for extemporaneous use

| | | |
|---|---|---|
| Active principle | g | 3 |
| Panthotenate calcium allantoine complex | g | 1 |
| Maltitol | g | 7 |

The mixture is thorougly mixed and the sieved through a fine mesh. The formulation is distributed in thermo-sealed envelopes containing 1 g each. The content of an envelope is dissolved in half glass of water before use.

C) Tooth-paste gel

| | | |
|---|---|---|
| Active principle | g | 0.6 |
| Carboxymethylcellulose medium viscosity | g | 6 |
| Pirogenic silica | g | 1 |
| Glycerine | g | 10 |
| Sodiumlaurylsulfate | g | 1 |
| Preserved water q.s. to | g | 100 |

D) Tooth-paste

| | | |
|---|---|---|
| Active principle | g | 0.6 |
| Calcium diphosphate | g | 35 |
| Glycerine | g | 30 |
| Sodium carboxymethylcellulose | g | 1 |
| Sodium laurylsulfate | g | 1.2 |
| Saccharine | g | 1.12 |
| Peppermint alcoholate | g | 1.5 |
| Water q.s. to | g | 100 |

The active principle is dissolved in water and sodium carboxymethylcellulose and saccharine are added thereto, stirring up to complete dispersion. Calcium diphosphate is kneaded with glycerine and the mixture is added under stirring to the previously prepared suspension. Laurylsulfate and the flavour are added and the mixture is distributed in plastified tube.

E) Gingival Paste

| | | |
|---|---|---|
| Active principle | g | 0.6 |
| Maltitol | g | 30 |
| Carboxymethylcellulose | g | 5 |
| Liquorice flavour | g | 0.1 |
| Peppermint flavour | g | 0.1 |
| Water q.s. to | g | 100 |

## Claims

**Claims for the following Contracting States : BE, CH, DE, FR, GB, LI, LU, NL, SE, AT**

1. Formulations for topical application in the oral cavity containing as active principles tromethamol (Mono-tris(hydroxymethyl)amino methane) or a salt thereof with non-toxic acid and pectic acid.

2. Formulations according to claim 1, wherein tromethamol is combined with pectic acid in the molar ratio from 0.5 to 1.5.

3. Formulations according to any one of the previous claims in form of tooth-pastes, mouth washes, gingival pastes, gengidentifrices (gum tooth-pastes), gum washes.

4. Use of tromethamol together with pectic acid in the preparation of a topical medicament for the oral hygiene having anticaries, antiplaque activity and for the care and prevention of pathologies of gums and of oral mucosa.

## Patentansprüche

**Patentansprüche für folgende Vertragsstaaten : BE, CH, DE, FR, GB, LI, LU, NL, SE, AT**

1. Formulierungen zur topischen Anwendung in der Mundhöhle, enthaltend Tromethamol (Tris-[hydroxymethyl]aminomethan) oder ein Salz derselben mit einer nicht-toxischen Säure und Pektinsäure als aktive Grundsubstanzen.

2. Formulierungen nach Anspruch 1, wobei Tromethamol mit Pektinsäure in einem Molverhältnis von 0,5 bis 1,5 kombiniert ist.

3. Formulierungen nach Anspruch 1 oder 2 in Form von Zahnpasten, Mundwässern, Gingivalpasten, Zahn- und Zahnfleischreinigern und Zahnfleischwäschen.

4. Verwendung von Tromethamol zusammen mit Pektinsäure zur Herstellung eines topischen Medikaments für die Mundhygiene, mit Antikaries- und Antiplaque-Aktivität und zum Schutz und zur Prävention gegen pathologische Zustände des Zahnfleisches und der Mundschleimhaut.

## Revendications

**Revendications pour les Etats conctractants suivants : BE, CH, DE, FR, GB, LI, LU, NL, SE, AT**

1. Formulations pour une application topique dans cavité buccale, contenant, comme principes actifs, du tromethamol (mono-tris(hydroxyméthyl)amino méthane) ou un sel de celui-ci avec un acide non-toxique et de l'acide pectique.

2. Formulations selon la revendication 1, dans lesquelles le tromethamol est combiné avec l'acide pectique dans le rapport molaire de 0,5 à 1,5.

3. Formulations selon l'une des revendications précédentes, sous la forme de pâtes dentifrices, d'eaux dentrifices, de pâtes gingivales, de dentifrices gingivaux (pâtes dentifrices pour gencives), d'eaux dentifrices pour gencives.

4. Utilisation du trométhamol conjointement avec de l'acide pectique dans la préparation d'un médicament topique pour l'hygiène buccale ayant une activité anti-caries, anti-plaque dentaire et pour les soins et la prévention des pathologies des gencives et de la mucose buccale.